# EUROPÄISCHE PATENTSCHRIFT

(11) **EP 2 038 263 B1**
(45) Veröffentlichungstag und Bekanntmachung des Hinweises auf die Patenterteilung: **05.03.2014**
(21) Anmeldenummer: 07787011.1
(22) Anmeldetag: 03.07.2007
(51) Int. Cl.: C07D 253/10, C07D 403/06, C07D 403/14, C07D 487/04, C07D 519/00, H01L 31/00

(54) **NEUE AROMATISCHE AZA-HETEROCYCLEN, HERSTELLUNGSVERFAHREN DAZU UND VERWENDUNG DES MATERIALS IN DER ORGANISCHEN ELEKTRONIK**
NOVEL AROMATIC AZA HETEROCYCLES, METHOD OF PRODUCTION AND USE OF THE MATERIAL IN ORGANIC ELECTRONICS
NOUVEAUX HÉTÉROCYCLES AZA-AROMATIQUES, PROCÉDÉ DE PRODUCTION ASSOCIÉ ET UTILISATION DU MATÉRIAU EN ÉLECTRONIQUE ORGANIQUE

(30) Priorität: 11.07.2006 DE 102006032107
(43) Veröffentlichungstag der Anmeldung: 25.03.2009
(73) Patentinhaber: OSRAM Opto Semiconductors GmbH, 93055 Regensburg (DE)
(72) Erfinder: ADLER, Jürgen, 91096 Kleinseebach (DE); KANITZ, Andreas, 91315 Höchstadt (DE)
(74) Vertreter: Epping - Hermann - Fischer
(86) Internationale Anmeldenummer: PCT/EP2007/056688
(87) Internationale Veröffentlichungsnummer: WO 2008/006738

(56) Entgegenhaltungen:
- US-A- 3 061 432
- US-A1- 2003 104 294

## Beschreibung

Die Erfindung betrifft ein neuartiges Elektronentransportmaterial auf der Basis annellierter aromatischer Elektronenmangelverbindungen.

Organische Halbleitermaterialien werden in Loch- und Elektronentransportmaterialien unterteilt. Diese werden beispielsweise zur Fertigung so genannter organischer elektronischer Bauelemente, wie Organische Leuchtdioden (OLEDs), Organischer Feldeffekttransistoren (OFETs), organischer Solarzellen, generell organischer photovoltaischer Elemente, elektrochromer organischer Bauteile, organischer Magnetsensoren, organischer Speicherelemente und/oder organischer Photodetektoren benötigt.

Auf der Seite der Lochtransportmaterialien wurden in den letzten 15 Jahren sehr effiziente und stabile Strukturen entwickelt, die je nach Anwendung mit unterschiedlichsten Lochinjektionseigenschaften zur Verfügung stehen und im lochtransportierenden, oxidierten Zustand stabile Radikalkationen bilden.

Auf der Seite der Elektronentransportmaterialien gibt es bisher nur sehr wenige Vertreter dieser Materialeigenschaft, sowohl in der Bandbreite der Elektroneninjektion als auch in der Stabilität dieser Materialien im elektronentransportierenden, reduzierten Zustand, so dass insbesondere die Radikalanionen über einen längeren Zeitraum nicht reversibel gebildet werden können.

Als gute Elektronenleiter gelten zur Zeit die Derivate des Phenanthrolin (BCP und BPhen) sowie Derivate des Oxadiazols. Die radikalanionschen Spezies, die während des Betriebes dieser Bauelemente gebildet werden, führen in den heterocyclischen Strukturen eine Geometrieänderung herbei, so dass die Elektronentransporteigenschaft als Folge der Ausbildung von Konjugationsunterbrechungen abnimmt.

Um dieses Problem zu beseitigen, war es Aufgabe dieser Erfindung effizientere Elektronenleiter zu entwickeln, die sich durch eine größere Injektionsbandbreite und vor allem durch die Fähigkeit zur Bildung reversibler Radikalanionen hoher Stabilität auszeichnen.

Gegenstand der Erfindung sind heteroaromatische Verbindungen, eine der folgenden heterocyclischen Stammstrukturen umfassend: Ein 1,2,4-Benzotriazin A und/oder B und/oder Pyrazolo-[1,5-a]-benzimidazol C und/oder D, 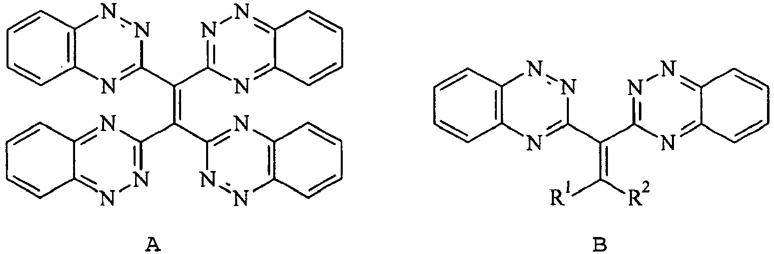 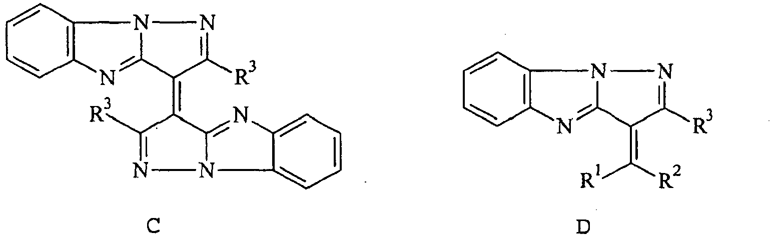 worin die Substituenten R¹ und R² voneinander unabhängig die Bedeutung Phenyl, 1-Naphthyl, 2-Naphthyl und/oder eines Aromaten, in dem die Elektronendichte im aromatischen Ring gegenüber einem Benzolring herabgesetzt ist, haben können. Aromaten, in denen die Elektronendichte im aromatischen Ring gegenüber dem Benzolring herabgesetzt ist werden auch als π-Unterschussaromaten bezeichnet. Insbesondere werden als π-Unterschussaromaten anellierte und nicht anellierte aromatische Sechsring-azaheteroyclen mit einem oder mehreren Stickstoffatomen verstanden.

R³ hat die Bedeutung von Aryl, insbesondere Phenyl sowie Al-kyl, insbesondere Methyl.

Außerdem ist Gegenstand der Erfindung die Herstellung dieser Verbindungen durch reduktive Cyclisierung von Nitroaromaten und schließlich ist Gegenstand der Erfindung die Verwendung dieser Verbindungen in organischen elektronischen Bauelementen, wie sie in der Einleitung oben beispielhaft aufgezählt sind.

Als π-Unterschussaromaten werden alle aromatischen Verbindungen bezeichnet, bei denen die Elektronendichte im aromatischen Ring gegenüber dem Benzolring herabgesetzt ist.

Diese entstehen entweder durch Substitution eines Kohlenstoff-Atoms durch ein elektronegativeres Atom (z. B Stickstoff, Schwefel, Sauerstoff) bei entsprechender Geometrie des Systems und/oder durch entsprechende elektronegative Substitution eines Wasserstoffatoms an einem Kohlenstoff des Grundgerüstes, wodurch die Elektronendichte im aromatischen Ring derart herabgesetzt wird, dass der aromatische Ring eine hohe Affinität zu einem weiteren Elektron hat. Insbesondere werden als π-Unterschussaromaten, anellierte und nicht anellierte aromatische Sechsring-azaheteroyclen mit einem oder mehreren Stickstoffatomen verstanden. Es gibt aber auch Fünfring π-Unterschussaromaten mit mindestens 3 Heteroatomen, insbesondere S, O und N, die beispielsweise an der Stelle eingesetzt werden können.

Zwar wurde schon vielfach versucht, Systeme zur Stabilisierung von Radikalanionen zu schaffen, was besonders durch Einführung von Lewissäuerederivaten ermöglicht wurde, jedoch sind diese Systeme, die im Grunde nur über die Methode der reduktiven Cyclisierung von Nitroaromaten zugänglich sind, bisher noch unerforscht geblieben.

Da in diesen Materialien konjugierte Anordnungen mit extrem starken π-Elektronenunterschussaromaten verknüpft wurden, sind diese auch in der Lage ein zusätzliches Elektron im π-System zu stabilisieren. Durch die Möglichkeit der Anordnung unterschiedlicher π-Unterschussheterocyclen in den Strukturtypen B und D kann die Injektionsbandbreite stark variiert werden.

Im Folgenden wird ein beispielhaftes Syntheseschema zur Herstellung der Verbindungen näher erläutert:
a) Synthese des Bis-(1,2,4-benzotriazin-3-yl)-methan-systems: (reduktive Cyclisierung) 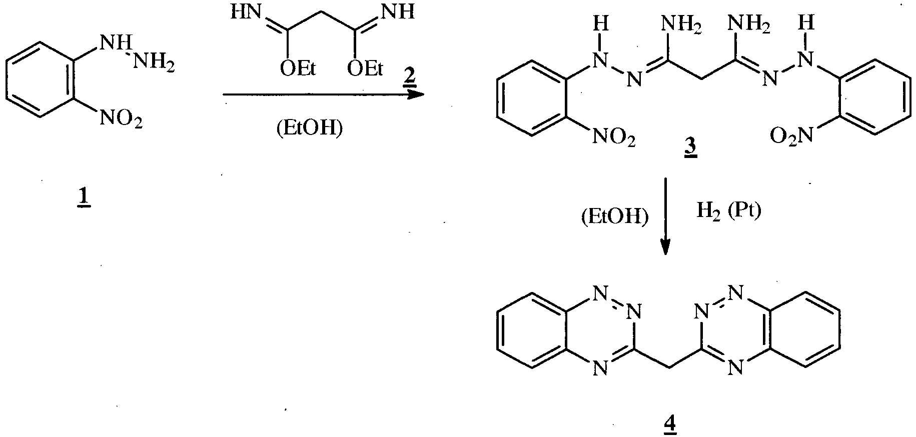
b) Synthese 3-substituierter Pyrazolo-[1,5-a]-benzimidazole:
   (reduktive Cyclisierung) 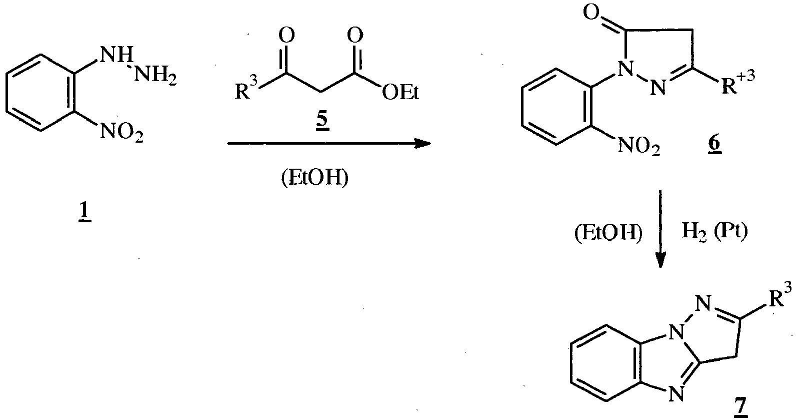
c) Synthese des Tetra-(1,2,4-benzotriazin-3-yl)-ethylen A: 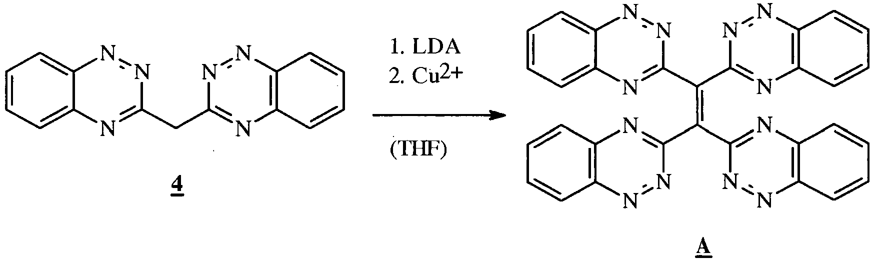
d) Synthese 3,3'-substituierter 4,4'Bis-(pyrazolyleno-[1,5-a]-benzimidazole C: 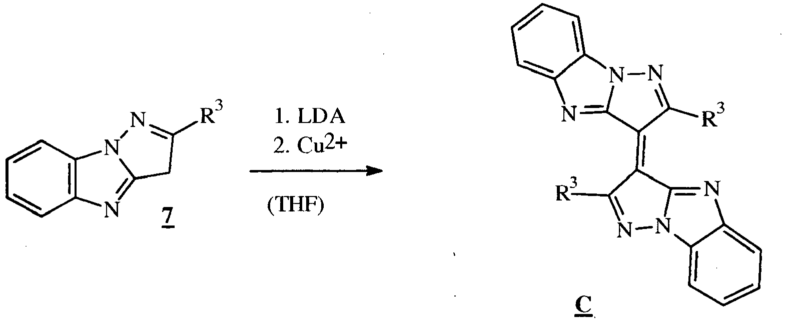
e) Synthese von 1,1-Diaryl-2,2-di-(1,2,4-benzotriazin-3-yl)-ethylenen B: 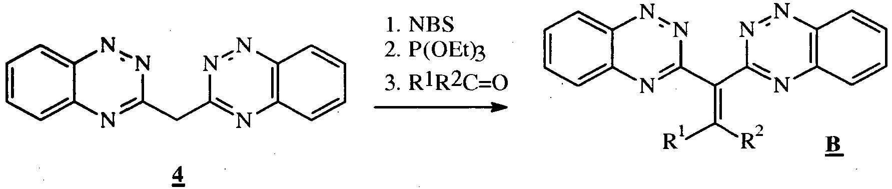
f) Synthese von 4-diarylmethylen-substituierten Pyrazolo-[1,5-a]-benzimidazolen D:

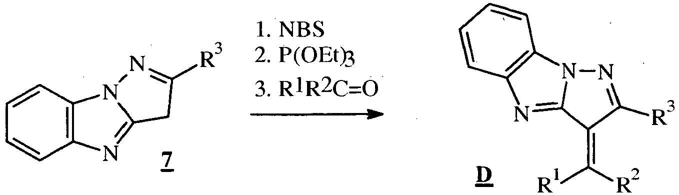

### Ausführungsbeispiele:

a) Synthese des Bis-amidrazon 3: 0,1mol o-Nitrophenylhydrazin 1 und 0,05mol Diiminomalonsäurediethylester 2 werden in 200ml Ethanol 4h am Rückfluss erhitzt. Nach Abkühlung wird das kristallisierte Bisamidrazon 3 abgesaugt, m.p.= °C, Ausbeute 70%d.T.
b) Synthese des Bis-(1,2,4-benzotriazin-3yl)-methan 4: In einer Hydrierapperatur werden 5g des Bisamidrazon 3 in 200ml Ethanol vorgelegt, mit einer Spatelspitze PtO₂ versetzt und anschließend bei Raumtemperatur unter Wasserstoffatmosphäre hydriert bis kein Wasserstoff mehr aufgenommen wird. Die entstandene gelbe Lösung wird vom Platinkontakt unter Luftausschluss getrennt und 2h unter Inertgas am Rückfluss erwärmt bis die Ammoniakabspaltung abgeschlossen ist. Danach wird die Lösung 2h mit Luftsauerstoff oxidiert. Die Lösung wird am Rotationsverdampfer eingeengt und zur Kristallisation gebracht.
c) Synthese der 1-(o-Nitrophenyl)pyrazolone 6: 0,1mol o-Nitrophenylhydrazin 1 und 0,12mol eines β-Ketoesters 4 werden in 200ml Ethanol 2h am Rückfluss erhitzt. Nach Abkühlung wird am Rotationsverdampfer um 2/3 eingeengt und das Pyrazolon 6 zur Kristallisation gebracht, m.p.= °C, Ausbeute 65% d.T.
d) Synthese der Pyrazolo-[1,5-a]-benzimidazole 7: In einer Hydrierapperatur werden 5g des Pyrazolon 6 in 200ml Ethanol vorgelegt, mit einer Spatelspitze PtO₂ versetzt und anschließend bei Raumtemperatur unter Wasserstoffatmosphäre hydriert bis kein Wasserstoff mehr aufgenommen wird. Die entstandene farblose Lösung wird vom Platinkontakt unter Luftausschluss getrennt und anschließend zur vollständigen Kondensation von 7 1h am Rückfluss erwärmt. Die Lösung wird am Rotationsverdampfer eingeengt und zur Kristallisation gebracht. Das Produkt ist ein farbloses amorphes Pulver, m.p.= °C, Ausbeute 55% d.T.
e) Synthese des Tetra-(1,2,4-benzotriazin-3-yl)-ethylen A: 2g Bis-(1,2,4-benzotriazin-3-yl)-methan 4 werden in 100ml trockenem THF in einer inerten Apperatur gelöst und mit der doppelten äquivalenten Menge LDA versetzt. Die dunkel gefärbte Lösung wird nun mit trockenem Kupfer(II) chlorid versetzt. Nach 30min Erwärmung wird das Gemisch am Rotationsverdampfer eingeengt, mit Wasser und verdünnter Salzsäure versetzt und schließlich mit Chloroform ausgeschüttelt. Die Chloroformphase wird nach dem trocknen eingeengt und das Produkt A mit Ethanol und Ether gefällt.
f) Synthese 3,3'-substituierter 4,4'Bis-(pyrazolyleno-[1,5-a]-benzimidazole C:
   2g Pyrazolo-[1,5-a]-benzimidazol 7 werden in 100ml trockenem THF in einer inerten Apperatur gelöst und mit der doppelten äquivalenten Menge LDA versetzt. Die dunkel gefärbte Lösung wird nun mit trockenem Kupfer(II)chlorid versetzt. Nach 30min Erwärmung wird das Gemisch am Rotationsverdampfer eingeengt, mit Wasser und verdünnter Salzsäure versetzt und schließlich mit Chloroform ausgeschüttelt. Die Chloroformphase wird nach dem trocknen eingeengt und das Produkt C mit Ethanol und Ether gefällt.
g) Synthese von 1,1-Diaryl-2,2-di-(1,2,4-benzotriazin-3-yl)-ethylenen B:
   0,01mol Bis-(1,2,4-benzotriazin-3-yl)-methan 4 wird in 50ml Tetra gelöst und mit der äquivalenten Menge NBS versetzt.
   Nach 6h erhitzen der Lösung am Rückfluss wird bei ca. 40°C abgesaugt und das Filtrat zur trockene eingeengt. Man versetzt den Rückstand mit äquivalenter Menge Triethylphosphit, 50ml Toluol und 1,5 Äquivalenten des gewünschten Diarylketon.
   Danach erhitzt man die Reaktionsmischung bei ca. 100°C im Ölbad und setzt der Lösung nach Erreichen der Ölbadtemperatur 2 Äqivalente Kaliumtert.-butylat zu. Nach weiteren 2h wird aufgearbeitet, indem die Mischung am Rotationsverdampfer vom Lösemittel befreit wird und durch Zusatz von Wasser, verdünnter Salzsäure und Chloroform das Produkt B extrahiert wird. Nach dem trocknen der Chloroformphase wird wiederum eingeengt und
   das Rohprodukt aus einer Mischung von Ethanol und Ether gefällt.
h) Synthese von 4-diarylmethylen-substituierten Pyrazolo-[1,5-a]-benzimidazolen D:
   0,01mol Pyrazolo-[1,5-a]-benzimidazol 7 wird in 50ml Tetra gelöst und mit der äquivalenten Menge NBS versetzt. Nach 6h erhitzen der Lösung am Rückfluss wird bei ca. 40°C abgesaugt und das Filtrat zur trockene eingeengt. Man versetzt den Rückstand mit äquivalenter Menge Triethylphosphit, 50ml Toluol und 1,5 Äquivalenten des gewünschten Diarylketon. Danach erhitzt man die Reaktionsmischung bei ca. 100°C im Ölbad und
   setzt der Lösung nach Erreichen der Ölbadtemperatur 2 Äquivalente Kaliumtert.-bütylat zu. Nach weiteren 2h wird aufgearbeitet, indem die Mischung am Rotationsverdampfer vom Lösemittel befreit wird und durch Zusatz von Wasser, verdünnter Salzsäure und Chloroform das Produkt D extrahiert wird. Nach dem trocknen der Chloroformphase wird wiederum eingeengt und
   das Rohprodukt aus einer Mischung von Ethanol und Ether gefällt.

Durch die hohe Radikalionenstabilität der Verbindungen nach der Erfindung wird erreicht, dass sich daraus hergestellte Materialien bestens als Elektronenleiter für organische e-lektronische Bauelemente einsetzen lassen. Die Substanzen sind in gängigen Lösungsmitteln löslich und lassen sich in druckfähige Pasten oder Dispersionen einbringen, so dass sie sich massenfertigungstauglich verarbeiten lassen.

Die Erfindung betrifft ein neues Elektronentransportmaterial auf der Basis annellierter aromatischer Elektronenmangelverbindungen. Durch die Anhäufung aromatischer Ringe mit Elektronenunterschuss lassen sich Radikalanionen in diesen Systemen bestens stabilisieren.

## Patentansprüche

1. Heteroaromatische Verbindung, eine der folgenden heterocyclischen Stammstrukturen umfassend: Ein 1,2,4-Benzotriazin A und/oder B und/oder Pyrazolo-[1,5-a]-benzimidazol C und/oder D, 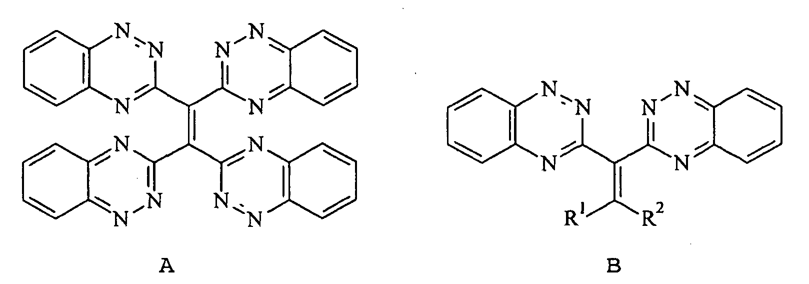 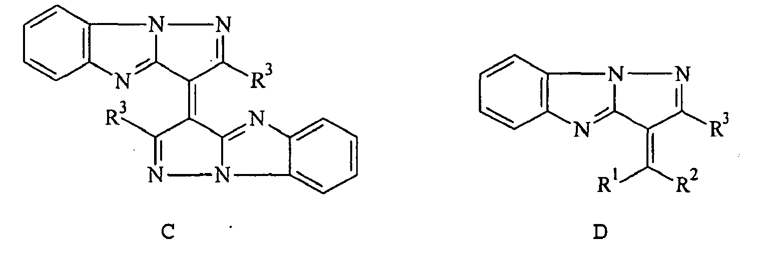 worin die Substituenten R¹ und R² voneinander unabhängig die Bedeutung Phenyl, 1-Naphthyl, 2-Naphthyl und/oder eines Aromaten, in dem die Elektronendichte im aromatischen Ring gegenüber einem Benzolring herabgesetzt ist, haben können; R³ hat die Bedeutung von Aryl und/oder Alkyl.

2. Verbindung nach Anspruch 1, bei dem R³ zumindest in einer Komponente die Bedeutung von Phenyl hat.

3. Verbindung nach Anspruch 1 oder 2, bei dem R³ zumindest in einmal die Bedeutung Methyl hat.

4. Verfahren zur Herstellung einer Verbindung nach einem der Ansprüche 1 bis 3, über reduktive Cyclisierung von Nitroaromaten.

5. Verwendung einer Verbindung nach einem der vorhergehenden Ansprüche als Elektronentransportmaterial in der organischen Elektronik.

6. organisches elektronisches Bauelement, das eine Verbindung nach einem der Ansprüche 1 bis 4 umfasst.

## Claims

1. Heteroaromatic compound comprising one of the following heterocyclic parent structures: a 1,2,4-benzotriazine A and/or B and/or pyrazolo-[1,5-a]-benzimidazole C and/or D, 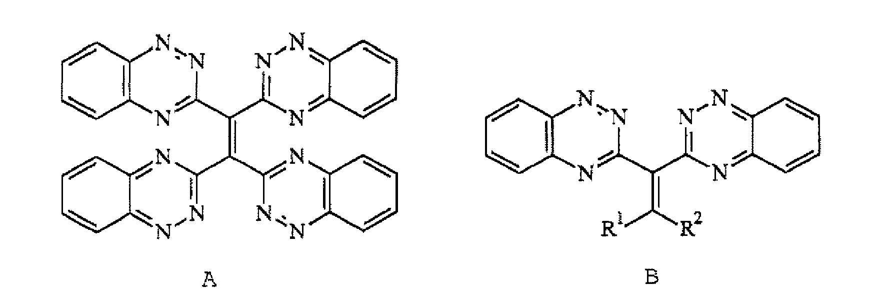 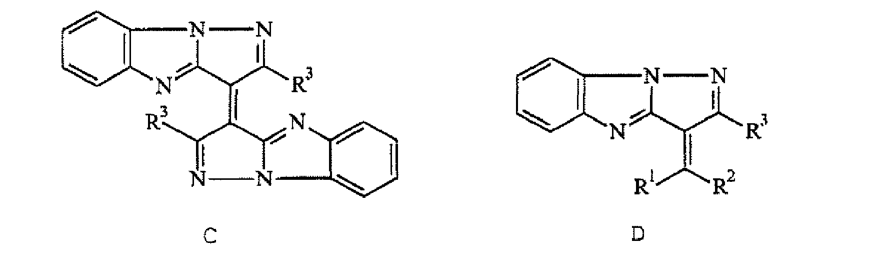 in which the substituents R¹ and R² may each be defined independently as phenyl, 1-naphthyl, 2-naphthyl and/or an aromatic in which the electron density in the aromatic ring is lowered with respect to a benzene ring; R³ is defined as aryl and/or alkyl.

2. Compound according to Claim 1, in which R³ at least in one component is defined as phenyl.

3. Compound according to Claim 1 or 2, in which R³ is at least once defined as methyl.

4. Process for preparing a compound according to any one of Claims 1 to 3 via reductive cyclization of nitroaromatics.

5. Use of a compound according to any one of the preceding claims as an electron transport material in organic electronics.

6. Organic electronic component which comprises a compound according to any one of Claims 1 to 4.

## Revendications

1. Composé hétéroaromatique, comprenant une des structures souches hétérocycliques suivantes : une 1,2,4-benzotriazine A et/ou B et/ou un pyrazolo-[1,5-a]-benzimidazole C et/ou D, 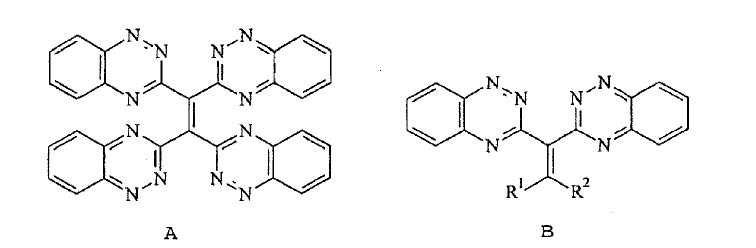 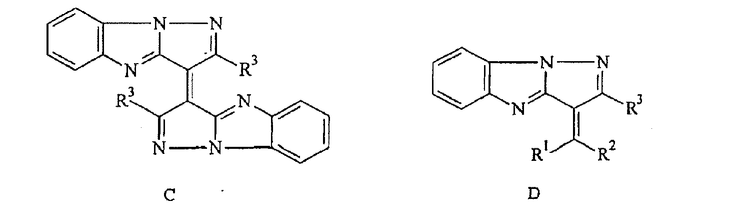 où les substituants R¹ et R² peuvent présenter, indépendamment l'un de l'autre, la signification phényle, 1-naphtyle, 2-naphtyle et/ou d'un aromatique, dans lequel la densité électronique dans le cycle aromatique est diminuée par rapport à un cycle benzène ; R³ présente la signification aryle et/ou alkyle.

2. Composé selon la revendication 1, dans lequel R³ présente, dans au moins un composant, la signification phényle.

3. Composé selon la revendication 1 ou 2, dans lequel R³ présente au moins une fois la signification méthyle.

4. Procédé pour la préparation d'un composé selon l'une quelconque des revendications 1 à 3 par cyclisation réductrice de nitroaromatiques.

5. Utilisation d'un composé selon l'une quelconque des revendications précédentes comme matériau de transport d'électrons dans l'électronique organique.

6. Module électronique organique, qui comprend un composé selon l'une quelconque des revendications 1 à 4.
